# EUROPEAN PATENT SPECIFICATION

(11) **EP 2 494 061 B1**
(45) Date of publication and mention of the grant of the patent: **25.01.2017**
(21) Application number: 10830418.9
(22) Date of filing: 25.10.2010
(51) Int. Cl.: C07K 16/00

(54) **ANTIBODY GLYCOSYLATION VARIANTS**
ANTIKÖRPERGLYCOSYLIERUNGSVARIANTEN
VARIANTES DE GLYCOSYLATION DES ANTICORPS

(30) Priority: 29.10.2009 US 255986 P
(43) Date of publication of application: 05.09.2012
(73) Proprietor: Janssen Biotech, Inc., Horsham, PA 19044 (US)
(72) Inventor: LUO, Jinquan, Radnor PA 19087 (US); MCCARTHY, Stephen, Radnor PA 19087 (US); RAJU, T., Shantha, Radnor PA 19087 (US); SCALLON, Bernard, Radnor PA 19087 (US); SPINKA-DOMS, Tracy, Radnor PA 19087 (US)
(74) Representative: Carpmaels & Ransford LLP
(86) International application number: PCT/US2010/053948
(87) International publication number: WO 2011/059684

(56) References cited:
- WO-A1-2009/045894
- WO-A2-2006/104989
- WO-A2-2007/005612
- WO-A2-2009/099961
- US-A- 5 041 376
- US-A- 5 225 354
- US-A1- 2007 041 979
- R. J. BREZSKI ET AL: "Tumor-associated and microbial proteases compromise host IgG effector functions by a single cleavage proximal to the hinge", PROCEEDINGS OF THE NATIONAL ACADEMY OF SCIENCES, vol. 106, no. 42, 20 October 2009 (2009-10-20), pages 17864-17869, XP055058828, ISSN: 0027-8424, DOI: 10.1073/pnas.0904174106

## Description

### BACKGROUND

### Field of the Invention

The invention relates to evaluating the Fc sequence of antibodies and other Fc-containing molecules and, more particularly, to methods of preparing, altering and using antibody preparations and other Fc-containing molecules to alter the susceptibility to proteases.

### Discussion of the Field

Amino acid modifications within the Fc domain may have what can be considered allosteric effects, that is, affecting Fc conformation from a distance. In particular, amino acid substitutions in the CH3 domain have been shown to affect binding to Fc-gamma receptors, which bind the antibody below the interchain disulfide bonds between heavy chains (the lower hinge region) which is also the CH2 domain (Shields et al. (2001) J Biol Chem 276:6591; Stavenhagen et al.(2007) Cancer Res 67:8882).

In the mature antibody, the two complex bi-antennary oligosaccharides attached to Asn297 are buried between the CH2 domains, forming extensive contacts with the polypeptide backbone. It has been found that their presence is essential for the antibody to mediate effector functions, such as ADCC (Lifely, M. R., et al., Glycobiology 5:813-822 (1995); Jefferis, R., et al., Immunol Rev. 163:59-76 (1998); Wright, A. and Morrison, S. L., supra). Studies by other and by the present applicants (WO2007005786) have further demonstrated that the oligosaccharide composition of these naturally appended glycans in the Fc region also alter Fc-receptor binding affinities and protease sensitivity in various nonadjacent sites of the polypeptide chain (Raju, S.T. 2008 Curr Op Immunol 20:471-478; WO2007024743). WO2009045894 describes methods and structural conformations of antibody preparations with increased resistance to proteases. US5041376 describes a method for identifying or shielding functional sites or epitopes of proteins that enter the exocytotic pathway of eukaryotic cells.

Thus, as the understanding of the various conformational aspects of antibody molecules evolves and modeling and protein engineering techniques become more sophisticated, it now becomes possible to target regions within therapeutic antibody candidates for modification to match the desired spectrum of *in vivo* interactions for a particular use or indication. Such modification may provide improved antibody therapeutics with retained safety.

### SUMMARY OF THE INVENTION

The invention provides an Fc-containing molecule comprising an modified antibody Fc domain amino acid sequence with N glycosylation sites at the ends of loop structures in the CH3 domain, wherein the Fc containing molecule has increased resistance to protease, as compared to Fc containing molecules having the analogous unmodified antibody Fc domain amino acid sequence.

The invention further provides a method of increasing resistance of an Fc-containing protein to cleavage by a protease, comprising adding N-glycosylation sites to the Fc-containing protein correlative to the EU numbering at at least one of positions 359, 382, and 419.

The present invention provides the compositions of modified, glycosylated immunoglobulin constant domains useful in engineering of antibody or antibody-like therapeutics, such as those comprising an Fc region, having one or more engineered Asn-linked glycosylation sites ("N-glycosylation").

In an embodiment of the invention, there is an N-glycosylation site at position 359 from a mutation at position 361 and/or an N-glycosylation site at position 419 from a mutation at position 421. Additionally, the native Fc glycosylation at Asn297 is present and in another embodiment the native Fc glycosylation may be absent. The antibody-derived constructs are dimeric protein structures derived from or comprising human IgG1, IgG2, IgG3, or IgG4 sequences. In one aspect, the constructs contain amino acid substitutions at positions 228, 234, or 235 (Kabat EU numbering) in the hinge region.

Another object of the invention comprises compounds based on the modified, glycosylated immunoglobulin constant domains with improved properties as compared to compounds having the analogous unmodified immunoglobulin constant domain; the properties including, but not limited to, protease sensitivity, serum half-life, and Fc-receptor binding.

It is a further object of the invention to provide compositions and methods for enhancing the ability of glycosylated antibody preparations to resist cleavage by proteases and therefore provide antibody preparations to treat pathological conditions associated with the presence of elevated levels of proteases, such as cancer. In yet another embodiment of the method, the glycosylated Fc-containing protein is an antibody, preferably a therapeutic monoclonal antibody. The protease, the cleavage activity of which is to be resisted, is selected from the group consisting of pepsin, plasmin, trypsin, chymotrypsin, a matrix metalloproteinase, a serine endopeptidase, and a cysteine protease, arising from the host or a pathogen which may be a parasite, bacterium or a virus. In a specific embodiment, the protease is a matrix metalloproteinase selected from the group consisting of gelatinase A (MMP2), gelatinase B (MMP-9), matrix metalloproteinase-7 (MMP-7), stromelysin (MMP-3), and macrophage elastase (MMP-12). The modifications can be introduced into antibody sequences. The disclosed modified constructs show greater resistance to physiologically-relevant proteases.

### BRIEF DESCRIPTION OF THE DRAWING

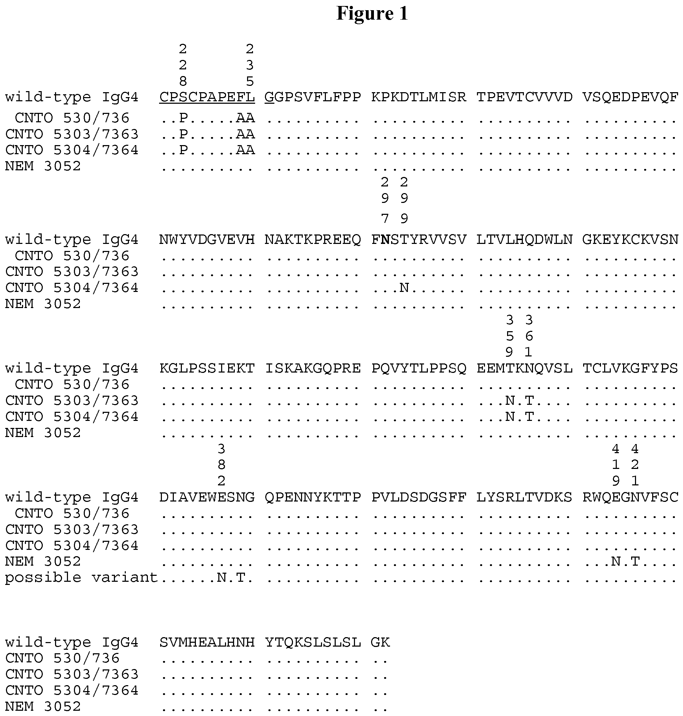

**Figure 1** shows an alignment of the amino acid sequences of hinge and Fc domains of IgG4-based variants and where the number is based on the EU antibody number of Kabat. The sequence shown begins with the core hinge (residue 227) and ends with the C-terminus of the Fc domain (residue 447) indicating that CNTO 5303 and CNTO 7363 differ from CNTO 530 and CNTO 736, respectively, by having an Asn (N) at position 359 instead of a Thr, and a Thr (T) at position 361 instead of an Asn, resulting in creation of glycosylation motif and causing the protein to be glycosylated at Asn359. The variants, CNTO 5304 and CNTO 7364, differ from CNTO 5303 and CNTO 7363 by having Thr at position 299 replaced with Asn, thereby removing the motif and glycosylation at Asn297. The NEM 3052 sequence, by changing amino acids at positions 419 and 421, results in glycosylation motif and glycosylation at position 419. Another site shown for a creation of glycosylation motif position is between 382 and 384 shown in the figure as ("possible variant"). Dots indicate the amino acid is the same as in the wild-type sequence.
**Figure 2** shows the structure of an Fc fragment residue 359, a site of new glycosylation, highlighted on both heavy chains.

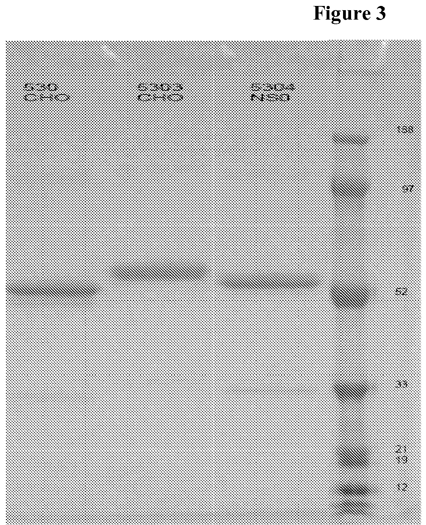

**Figure 3** shows CNTO 530 and its variants fractionated through an SDS-PAGE gel (nonreduced)
**Figure 4** shows an AlphaScreen-based analysis of how well the two MIMETIBODY™ construct variants compete with a biotinylated mAb for binding to human FcRn.
**Figures 5A-F** shows data derived for MALDI-TOF-MS tracings of the rate of disappearance of the intact Fc-constructs upon incubation with human MMP-3 or human neutrophil elastase (NE) over time A-D) CNTO 5303 to CNTO 530, and comparison of CNTO 7363 to CNTO 736, when incubated with MMP-3 or NE. E, F) all samples, including CNTO 5304 and CNTO 7364, when incubated with the two proteases.

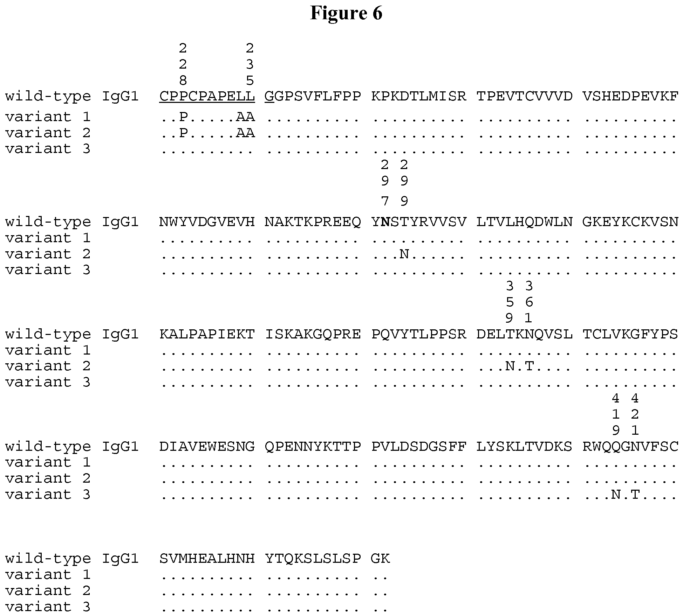

**Figure 6** shows the amino acid sequences of hinge and Fc domains of IgG1-based variants as in Fig. 1.
**Figure 7** is a graph depicting the serum persistence of CNTO530 vs. CNTO5303 in the blood of mice injected with both molecules.

### DETAILED DESCRIPTION OF THE INVENTION

### Abbreviations

AA = anthranilic acid; α1,3GT = α-1,3-galactosyltransferase; ARD = acute respiratory distress; β1,4GT = β-1,4-galactosyltransferase; α2,3ST = α-2,3-sialyltransferase; ADCC = antibody-dependent cellular cytotoxicity; CDC = complement-dependent cytotoxicity; CMP-Sia = cytidine monophosphate *N*-acetylneuraminic acid; FBS = fetal bovine serum; IgG = immunoglobulin G; MALDI-TOF-MS = matrix-assisted laser/desorption ionization time-of-flight mass spectrometry; NANA = *N*-acetylneuraminic acid isomer of sialic acid; NGNA = *N-*glycolylneuraminic acid isomer of sialic acid; OA = osteoarthritis; PNGase F = peptide *N-*glycosidase F; HPLC = reversed phase high-performance liquid chromatography; RA = rheumatoid arthritis; SA = Sinapic acid; Sia = sialic acid; SDHB = dihydroxybenzoic acid containing sodium chloride; UDP-Gal = uridine diphosphate galactose; UDP-GlcNAc = uridine diphosphate *N*-acetylglucosamine.

### Definitions & Explanation of Terminology

The terms "Fc," "Fc-containing protein" or "Fc-containing molecule" as used herein refer to a monomeric, dimeric or heterodimeric protein having at least an immunoglobulin CH2 and CH3 domain. The CH2 and CH3 domains can form at least a part of the dimeric region of the protein/molecule (e.g., antibody).

The term "antibody" is intended to encompass antibodies, digestion fragments, specified portions and variants thereof, including, without limitation, antibody mimetics or comprising portions of antibodies that mimic the structure and/or function of an antibody or specified fragment or portion thereof, including, without limitation, single chain antibodies, single domain antibodies, minibodies, and fragments thereof. Functional fragments include antigen-binding fragments that bind to the target antigen of interest. For example, antibody fragments capable of binding to a target antigen or portions thereof, including, but not limited to, Fab (e.g., by papain digestion), Fab' (e.g., by pepsin digestion and partial reduction) and F(ab')₂ (e.g., by pepsin digestion), facb (e.g., by plasmin digestion), pFc' (e.g., by pepsin or plasmin digestion), Fd (e.g., by pepsin digestion, partial reduction and reaggregation), Fv or scFv (e.g., by molecular biology techniques) fragments, are encompassed by the term antibody (see, e.g., Colligan, Immunology, supra).

The term "monoclonal antibody" as used herein is a specific form of Fc-containing fusion protein comprising at least one ligand binding domain which retains substantial homology to at least one of a heavy or light chain antibody variable domain of at least one species of animal antibody.

### Overview

The present invention was spurred by an interest in identifying a new site on an Fc domain for PEGylation. As techniques are known for conjugation of PEG moieties to the glycans of proteins, which provides a specific targeting site for the modification, the use of the natural glycans at Asn297 was attemped, however, due to the tertiary and quaternary structure of the Fc-dimeric structure, the native Fc glycans have been shown to be insufficiently accessible to enable conjugations of large PEG structures.

Therefore, positions on the Fc domain were considered where an alternate or additional N-linked glycosylation site could be introduced by engineering in the motif sequence Asn-Xxx-Ser/Thr, known as a recognition site for glycosyltransferases in the endoplasmic reticulum of eukaryotic cells. In making such glycan variants for two different Fc-comprising constructs, CNTO 530 (EPO MIMETIBODY™ construct) and CNTO 736 (GLP-1 MIMETIBODY™ construct), it was observed that the non-PEGylated glycan variants unexpectedly showed significantly increased resistance to proteolytic enzymes.

The body naturally produces proteases for digestion and remodeling of proteins, to which therapeutic proteins are also subjected. In non-pathogen driven disease states, such as RA and other inflammatory diseases, and cancer, it is well known that a certain spectrum of proteolytic enzymes are elevated. Also, it is well-known that human proteases are associated with inflammatory, proliferative, metastatic, and infectious diseases. Circulating immunoglobulins, and specifically those antibodies of the IgG class, are major serum proteins. It has been appreciated that human proteases, matrix metalloproteinases (MMPs) and neutrophil elastase, cleave the IgG heavy chain polypeptide at a residue unique to each protease similar to bacterial proteases, such as glutamyl endopeptidase (*Staph. aureus*) or immunoglobulin degrading enzyme of streptococcus (*Strep. pyogenes*). The cleavage sites in the heavy chain are clustered around the region termed the hinge domain, where the interchain disulfide linkage of the two heavy chains occurs. The region below the hinge constitutes the Fc region and comprises binding sites responsible for the effector functions of IgG. In the case of microorganisms, protease expression is a potential adjunctive virulence pathway allowing organisms to avoid opsonization (Rooijakkers et al. Microbes and Infection 7: 476-484, 2005) in so far as the proteolytic release of the Fc domain by cleavage below the hinge effectively neutralizes functions that would otherwise lead to the targeting and killing of that pathological cell. Thus, the elaboration of specific proteases may be representative of a myriad of diseases states including cancer, inflammation and infectious diseases. That IgG degradation is enhanced in pathologic *in vivo* environments is further evidenced by the presence of natural IgG autoantibodies that bind to the cleaved hinge domain (Knight et al., 1995; Nasu et al., 1980; Persselin and Stevens, 1985, Temess, et al. 1995 J Imunol. 154: 6446-6452). Thus, the increased resistance to physiologically-relevant proteases could result in a prolonged in vivo half-life for therapeutic Fc-containing molecules, particularly in protease-rich environments, which could enhance efficacy and/or enable less-frequent dosing.

A commonly owned patent application, WO2009/023457, discloses proteases capable of degrading IgG and which are associated with disease or pathological states, such as cancer, inflammation, and infection. The information is summarized in Table 1 (reproduced below), in which "Coagulation proteinases" included F.XIIa, FIXa, F.Xa, thrombin and activated protein C; plasmin was plasminogen co-incubated with plasminogen activators; tPA, streptokinase and staphylokinase; "plasminogen activators alone" are without plasminogen; and the MMPs were recombinant proteinases obtained either as the active form or the pro-enzyme; and "None" denotes no detectable cleavage in 24 hours. Except where indicated, all enzymes were human. The residue designations are for the EU numbering system for the complete mature IgG1 antibody heavy chain.

**Table 1.**

| Enzyme | Source | Proteinase Type | Disease Association (Ref) | Cleaved Site | Major Product |
|---|---|---|---|---|---|
| Cathepsin G | Human Neutrophil granules | Serine endopeptidase | Emphysema, IPF, RA (2,3) | Glu²³³-leu²³⁴ | F(ab')₂ + Fc |
| Cathepsin B | " | " | | | None |
| Cathepsin D | " | " | | | None |
| Neutrophil elastase (HNE, leukocyte elastase, PMN elastase) | " neutrophils | " | Amyloidosis, lung emphysema, cystic fibrosis, ARDS, RA, tumor invasion (2,3) | Thr²²³-his²²⁴ | Fab + Fc |
| Pancreatic elastase | | | Pancreatititis (3) | | |
| Proteinase 3 (myeloblastin) | " | " | | | None |
| Tryptase | " mast cells | " | Anaphylaxis, fibrosis (2) | | None |
| Chymase | " mast cells | " | Inflammation, cardiovascular diseases (2, 3) | | None |
| Kallekrein | " | " | | | None |
| Coagulation proteinases | " | " | | | None |
| Plasmin (fibrinolysin) | " | " | Cell migration (e.g.tumors)(2) Streptococcal infection (6) | Lys²²³-thr²²⁴ | Fab + Fc |
| Plasminogen activators alone | " | " | | | None |
| Interstitial collagenase (MMP-1) | Human (fibroblasts , chondrocyt es) | Metalloendopeptidase | RA, OA, IBD, IPF, aneurysms (1) | | None |
| Gelatinase A (MMP-2) | " tumor cells, fibroblasts | " | Invasive tumors (1) | Glu²³³-leu²³⁴ | F(ab')₂ + Fc |
| Stromelysin (MMP-3) | " fibroblasts, chondrocyt es, osteoclasts, macrophages | " | RA, OA, atherosclerotic plaque, Crohn's disease, colitis, some tumors (1, 4) | Glu²³³-leu²³⁴ | F(ab')₂ + Fc |
| Matrilysin (MMP-7) | " glandular epithelial cells | " | Invasive tumors (1, 4) | Leu²³⁴-leu²³⁵ | F(ab')₂ + Fc |
| Collagenase 2 (MMP-8) | " neutrophils | | Inflammation, RA, OA (1, 4) | | None |
| Gelatinase B (MMP-9) | " normal and tumor cells, activated monocytes, neutrophils , T cells | " | Inflammation, aortic aneurysms, ARDS, bums RA > OA, inflammatory cell tumor infiltrates (1, 4) | Leu²³⁴-leu²³⁵ | F(ab')₂ + Fc |
| Macrophage metalloelastase (MMP-12) | " macrophag es | " | Inflammation, tissue destruction when over-expressed, aneurysms, atherosclerotic plaque (1) | Pro²³²-glu²³³ | F(ab')₂ + Fc |
| Cathepsin S | " | Cysteine endopeptidase | | | None |
| Glutamyl endopeptidase I (Glu V8 protease) | *Staph. aureus* | Serine endopeptidase | *Staph. Aureus* infection (2) | Glu²³³-leu²³⁴ | F(ab')₂+ Fc |
| Immunoglobulin degrading Enzyme of Streptococcus (IdeS) | *Strep. Pyogenes* | Serine endopeptidase | *Strep. Pyogenes* infection (5) | Gly²³⁶-gly²³⁷ | F(ab')₂ + Fc |

| | | | | | |
|---|---|---|---|---|---|
| (1) Barrett A. J., Rawlings N. D. and Woessner J. F.(Eds.), Handbook of Proteolytic Enzymes Vol. 1, Elsevier, Amsterdam, 2004. (2) Barrett A. J., Rawlings N. D. and Woessner J. F.(Eds.), Handbook of Proteolytic Enzymes Vol. 2, Elsevier, Amsterdam, 2004. (3) Powers, JC., "Proteolytic Enzymes and Disease Treatment" 1982. In: Feeney and Whitaker (eds). Modification of Proteins: Food, Nutritional, and Pharmacological Aspects. Advances in Chemistry Series 198. ACS, Washington, D.C. 1982 pp 347-367. (4) Tchetverikov I., Ronday H. K., van El B., Kiers G. H., Verzijl N., TeKoppele J. M., Huizinga T. W. J., DeGroot J. and Hannemaaijer R., 2004. MMP Profile in paired serum and synovial fluid samples of patients with rheumatoid arthritis. Ann.Rheum.Dis. 63, 881-883. (5) Vincents B., von Pawel-Rammingen U., Björck L. and Abrahamson M., 2004. Enzymatic characterization of the streptococcal endopeptidase, IdeS, reveals that it is a cysteine protease with strict specificity for IgG cleavage due to exosite binding. Biochemistry 43, 15540-15549. (6) Sun H., Ringdahl U., Homeister J.W., Fay W.P., Engleberg N.C., Yang A.Y., Rozek L.S., Wang X., Sjobring U., Ginsburg D., 2004. Plasminogen is a critical host pathogenicity factor for group A streptococcal infection. Science. 305, 1283-1286. | | | | | |

### Oligosaccharide Functions

Specific oligosaccharides are present on secreted proteins as a result glycosylation which takes place in the endoplasmic reticulum of eukaryotic cells as the normal processing of proteins designated by signal sequences for export from the cell. The oligosaccharide composition appended to the protein is affected by factors, such as the nature of the protein, the species of origin of the cell, the culture conditions, and the extracellular milieu. The nature of the "glycome" from species to species or even individual to individual has long been recognized as the source of antigenic epitopes, e.g., the human blood groups. Thus, protein surface glycosylation represents a method to alter recognition of proteins by targeting specific or nonspecific receptors for particular glycan structures or terminal saccharides. Oligosaccharides or ligands for mammalian receptors similar to lectins, such as the selectins, e.g. mannose-binding proteins, L-selectin, and P-selectin.

The glycans normally appended to the Asn 297 of the CH2 domain in mammalian IgG molecules act to provide tertiary structure for the Fc, two polypeptide chains covalently linked at the hinge region about the CH2 domain and by noncovalent association of the two CH3 domains. Aglycosylated IgG do not bind Fc-receptors or exhibit the effector functions of ADCC or CDC or bind complement Clq. Recent studies (Kaneko, 2006 Science 313: 670-673; Shields et al., 2002 J Biol. Chem. 277:30 26733-26740) have demonstrated that Asn297 linked glycan content may also affect the affinity of binding of IgG molecules to Fc(gamma) receptors.

A preparation of human gamma globlulin, known as IVIG, has long been used as a general anti-inflammatory treatment. Recent studies in a murine serum-induced arthritis model where treatment with high-dose human IVIG suppresses arthritis, showed that prior enzymatic desialylation of IVIG abrogated its therapeutic benefit, whereas enrichment for the sialylated fraction of IVIG enhanced its anti-inflammatory benefit (Kaneko, 2006 Science 313: 670-673).

It was long known that the anti-inflammatory property is determined by the Fc portion of the IVIG. Subsequent work demonstrated that the fraction of IVIG molecules primarily responsible for suppressing joint inflammation in a murine arthritis model are those with Fc sialic acid in an α2,6 linkage with galactose as opposed to those with sialic acid in α2,3 linkage (Anthony et al., (2008) Science 320:373). The mouse lectin, SIGN-R1, expressed on the surface of splenic macrophages, is a receptor for α2,6 sialylated Fc fragments as is the human lectin, DC-SIGN expressed on human dendritic cells (Anthony, et al. Proc Natl Acad Sci USA. 2008 Dec 16;105(50):19571-8.).

Thus, using the protein compositions of the present invention, protein compositions having specified oligosaccharide structures, termini, or content can be synthesized via host cell manipulation and glycoengineering, or prepared by pre- or post-protein purification processing, such as fraction using lectin-affinity chromatography or enzymatic treatments or combinations of several methods. Such methods are known to those skilled in the art as taught herein or are being or can be developed using known methods in genetic engineering, enzymology, protein fraction, and the like. These preparations can be used to target specific receptors as they occur on selected cell types, tissues, or organs.

The glycosylation or hyperglycosylation of proteins increases the hydrated volume of a protein and can add negative charge due to the presence of sialic acid residues. These alterations render proteins less subject to clearance by kidney filtration. Thus, in addition to FcRn binding as a means by which the Fc fragment enhances protein half-life in the circulation, the increased circumference of the protein will produce an added effect, provided that additional glycosylation does not reduce FcRn binding.

### Method of Making the Altered Fc-Containing Molecules

The sites for additional glycosylation were chosen based on the desire was to add Asn-linked glycans without affecting the Fc structure or function. The IgG4 Fc structure (1adq) (Corper et al (1997) Nat Struct Biol. 4: 374) was analyzed to identify potential sites of modification. Loop regions of the CH3 domain distant from the Fc(gamma)R binding site in the lower hinge, and distant from the FcRn binding site at the CH2-CH3 junction region were targeted. The 359-TKNQVS-364, 382-ESNGQP-387, and 419-EGNVFS-424 loops contain residues that would appear to be amenable to modification. Within these loops, residues 359, 382, and 419 were identified as attractive sites to introduce glycosylation based on being surface exposed and based on predictions that an Asn substitution with resulting glycosylation would be structurally compatible. Then, a number of N-glycosylation sequence motifs (N X S/T) was computationally created for these positions and estimated their potential for glycosylation by submitting the sequences to the NetNGlyc server (www.cbs.dtu.dk/services/NetNGlyc). Motifs with a score of 0.5 or lower were eliminated. The motifs chosen for introduction into test molecules were 359NKT and 419NGT (Figure 1). The 382 site was not pursued due to the consideration that the new glycan may point in a direction that would interfere with FcRn binding. However, it is possible that introduction of a glycosylation site at residue 382 would have yielded a fully functional Fc domain.

### Enzymatic Modification of Fc-containing proteins

One method for preparing an Fc-containing protein with specific glycan structure or specified oliogsaccharride content is by treating the Fc-containing protein preparation with a saccharase, such as a fucosidase or sialidase enzyme, thereby removing specific sugar residues, e.g., fucose or sialic acids. Addition of saccharides to the Fc region can also be achieved using in vitro glycosylation methods.

Glycosyltransferases naturally function to synthesize oligosaccharides. They produce specific products with excellent stereochemical and regiochemical geometry. The transfer of glycosyl residues results in the elongation or synthesis of an oligo- or polysaccharide. A number of glycosyltransferase types have been described, including sialyltransferases, fucosyltransferases, galactosyltransferases, mannosyltransferases, N-acetylgalactosaminyltransferases, N-acetylglucosaminyltransferases and the like. Glycosyltransferases which are useful in the present invention include, for example, α-sialyltransferases, α-glucosyltransferases, α-galactosyltransferases, α-fucosyl- transferases, α-mannosyltransferases, α-xylosyltransferases, α-N-acetylhexosaminyltransferases, β-sialyltransferases, β-glucosyltransferases, β-galactosyltransferases, β-fucosyltransferases, β-mannosyltransferases, β-xylosyltransferases, and β-N-acetylhexosaminyltransferases, such as those from Neisseria meningitidis, or other bacterial sources, and those from rat, mouse, rabbit, cow, pig, human and insect and viral sources. Preferably, the glycosyltransferase is a truncation variant of glycosyltransferase enzyme in which the membrane-binding domain has been deleted. Exemplary galactosyltransferases include α(1,3) galactosyltransferase (E.C. No. 2.4.1.151, see, e.g., Dabkowski et al., Transplant Proc. 25:2921 (1993) and Yamamoto et al. Nature 345:229-233 (1990)) and α(1,4) galactosyltransferase (E.C. No. 2.4.1.38). Other glycosyltransferases can be used, such as a sialyltransferase.

An α(2,3)sialyltransferase, often referred to as the sialyltransferase, can be used in the production of sialyl lactose or higher order structures. This enzyme transfers sialic acid (NeuAc) from CMP-sialic acid to a Gal residue with the formation of an α-linkage between the two saccharides. Bonding (linkage) between the saccharides is between the 2- position of NeuAc and the 3-position of Gal. An exemplary α(2,3)sialyltransferase referred to as α (2,3)sialyltransferase (EC 2.4.99.6) transfers sialic acid to the non- reducing terminal Gal of a Galβ1→3Glc disaccharide or glycoside. See, Van den Eijnden et al., J. Biol. Chem., 256:3159 (1981), Weinstein et al., J. Biol. Chem., 257:13845 (1982) and Wen et al., J. Biol. Chem., 267:21011 (1992). Another exemplary α-2,3- sialyltransferase (EC 2.4.99.4) transfers sialic acid to the non-reducing terminal Gal of the disaccharide or glycoside. See, Rearick et al., J. Biol. Chem., 254:4444 (1979) and Gillespie et al., J. Biol. Chem., 267:21004 (1992). Further exemplary enzymes include Gal-β-1,4- GlcNAc α-2,6 sialyltransferase (See, Kurosawa et al. Eur. J. Biochem. 219: 375-381 (1994)).

Other glucosyltransferases particularly useful in preparing oligosaccharides of the invention are the mannosyltransferases including α(1,2) mannosyltransferase, α(1,3) mannosyltransferase, β(1,4) mannosyltransferase, Dol-P-Man synthase, OCh1, and Pmt1. Still other glucosyltransferases include N-acetylgalactosaminyltransferases including α(1,3) N-acetylgalactosaminyltransferase, β(1,4) N- acetylgalactosaminyltransferases (Nagata et al. J. Biol. Chem. 267:12082- 12089 (1992) and Smith et al. J. Biol Chem. 269:15162 (1994)) and polypeptide N-acetylgalactosaminyltransferase (Homa et al. J. Biol Chem. 268:12609 (1993)). Suitable N-acetylglucosaminyltransferases include GnTI (2.4.1.101, Hull et al., BBRC 176:608 (1991)), GnTII, and GnTIII (Ihara et al. J. Biolchem. 113:692 (1993)), GnTV (Shoreiban et al. J. Biol. Chem. 268: 15381 (1993)).

For those embodiments in which the method is to be practiced on a commercial scale, it can be advantageous to immobilize the glycosyl transferase on a support. This immobilization facilitates the removal of the enzyme from the batch of product and subsequent reuse of the enzyme. Immobilization of glycosyl transferases can be accomplished, for example, by removing from the transferase its membrane-binding domain, and attaching in its place a cellulose-binding domain. One of skill in the art will understand that other methods of immobilization could also be used and are described in the available literature.
Because the acceptor substrates can essentially be any monosaccharide or oligosaccharide having a terminal saccharide residue for which the particular glycosyl transferase exhibits specificity, substrate may be substituted at the position of its non-reducing end. Thus, the glycoside acceptor may be a monosaccharide, an oligosaccharide, a fluorescent-labeled saccharide, or a saccharide derivative, such as an aminoglycoside antibiotic, a ganglioside, or a glycoprotein including antibodies and other Fc-containing proteins. In one group of preferred embodiments, the glycoside acceptor is an oligosaccharide, preferably, Galβ(1-3)GlcNAc, Galβ(1-4)GlcNAc, Galβ(1- 3)GalNAc, Galβ(1-4)GalNAc, Man α(1,3)Man, Man α(1,6)Man, or GalNAcβ(1-4)-mannose. In a particular preferred embodiment, the oligosaccharide acceptor is attached to the CH2 domain of an Fc-containing protein.

The use of activated sugar substrate, i.e., sugar-nucleoside phosphate, can be circumvented by either using a regenerating reaction concurrently with the glycotransferase reaction (also known as a recycling system). For example, as taught in, e.g., U.S. Pat. 6,030,815, a CMP-sialic acid recycling system utilizes CMP-sialic acid synthetase to replenish CMP-sialic acid (CMP-NeuAc) as it reacts with a sialyltransferase acceptor in the presence of a α(2,3)sialyltransferase to form the sialyl-saccharide. The CMP-sialic acid regenerating system useful in the invention comprises cytidine monophosphate (CMP), a nucleoside triphosphate (for example, adenosine triphosphate (ATP), a phosphate donor (for example, phosphoenolpyruvate or acetyl phosphate), a kinase (for example, pyruvate kinase or acetate kinase) capable of transferring phosphate from the phosphate donor to nucleoside diphosphates and a nucleoside monophosphate kinase (for example, myokinase) capable of transferring the terminal phosphate from a nucleoside triphosphate to CMP. The α(2,3)sialyltransferase and CMP- sialic acid synthetase can also be viewed as part of the CMP-sialic acid regenerating system as removal of the activated sialic acid serves to maintain the forward rate of synthesis. The synthesis and use of sialic acid compounds in a sialylation procedure using a phagemid comprising a gene for a modified CMP-sialic acid synthetase enzyme is disclosed in international application WO 92/16640, published October 1, 1992.

An alternative method of preparing oligosaccharides is through the use of a glycosyltransferase and activated glycosyl derivatives as donor sugars, obviating the need for sugar nucleotides as donor sugars as taught in U.S. Pat. 5,952,203. The activated glycosyl derivatives act as alternates to the naturally-occurring substrates, which are expensive sugar-nucleotides, usually nucleotide diphosphosugars or nucleotide monophosphosugars in which the nucleotide phosphate is α-linked to the 1-position of the sugar.

Activated glycoside derivatives which are useful include an activated leaving group, such as, for example, fluoro, chloro, bromo, tosylate ester, mesylate ester, triflate ester and the like. Preferred embodiments of activated glycoside derivatives include glycosyl fluorides and glycosyl mesylates, with glycosyl fluorides being particularly preferred. Among the glycosyl fluorides, α-galactosyl fluoride, α-mannosyl fluoride, α-glucosyl fluoride, α- fucosyl fluoride, α-xylosyl fluoride, α-sialyl fluoride, alpha-N-acetylglucosaminyl fluoride, α-N-acetylgalactosaminyl fluoride, β-galactosyl fluoride, β-mannosyl fluoride, β-glucosyl fluoride, β-fucosyl fluoride, β-xylosyl fluoride, beta-sialyl fluoride, β-N-acetylglucosaminyl fluoride and β-N-acetylgalactosaminyl fluoride are most preferred.

Glycosyl fluorides can be prepared from the free sugar by first acetylating the sugar and then treating it with HF/pyridine. Acetylated glycosyl fluorides may be deprotected by reaction with mild (catalytic) base in methanol (e.g., NaOMe/MeOH). In addition, many glycosyl fluorides are commercially available. Other activated glycosyl derivatives can be prepared using conventional methods known to those of skill in the art. For example, glycosyl mesylates can be prepared by treatment of the fully benzylated hemiacetal form of the sugar with mesyl chloride, followed by catalytic hydrogenation to remove the benzyl groups.

A further component of the reaction is a catalytic amount of a nucleoside phosphate or analog thereof. Nucleoside monophosphates which are suitable for use in the present invention include, for example, adenosine monophosphate (AMP), cytidine monophosphate (CMP), uridine monophosphate (UMP), guanosine monophosphate (GMP), inosine monophosphate (IMP) and thymidine monophosphate (TMP). Nucleoside triphosphates suitable for use in accordance with the present invention include adenosine triphosphate (ATP), cytidine triphosphate (CTP), uridine triphosphate (UTP), guanosine triphosphate (GTP), inosine triphosphate (ITP) and thymidine triphosphate (TTP). A preferred nucleoside triphosphate is UTP. Preferably, the nucleoside phosphate is a nucleoside diphosphate, for example, adenosine diphosphate (ADP), cytidine diphosphate (CDP), uridine diphosphate (UDP), guanosine diphosphate (GDP), inosine diphosphate (IDP) and thymidine diphosphate (TDP). A preferred nucleoside diphosphate is UDP. As noted above, the present invention can also be practiced with an analog of the nucleoside phosphates. Suitable analogs include, for example, nucleoside sulfates and sulfonates. Still other analogs include simple phosphates, for example, pyrophosphate.

One procedure for modifying recombinant proteins produced, in e.g., murine cells wherein the hydroxylated form of sialic acid predominates (NGNA), is to treat the protein with sialidase, to remove NGNA-type sialic acid, followed by enzymatic galactosylation using the reagent UDP-Gal and betal,4 Galtransferase to produce highly homogeneous G2 glycoforms. The preparation can then, optionally, be treated with the reagent CMP-NANA and alpha-2,3 sialyltransferase to give highly homogeneous G2S2 glycoforms.

For purposes of this invention, substantially homogeneous for a glycoform shall mean about 85% or greater of that glycoform and, preferably about 95% or greater of that glycoform.

### Proteases and Protease Sensitivity of Antibodies

Pepsin is auto-activated and active at low pH as it is a normal component of the gastric fluid secreted into the lumen of the stomach after eating. Low levels of the precursor enzyme pepsinogen can be found in the serum but, since activation and activity are acid dependent, is not physiologically relevant to circulating antibodies. Pepsin cleaves human IgG1 between the leucine₂₃₄-leucine₂₃₅ in the lower hinge. This cleavage site is downstream from the hinge core (-C-P-P-C-) containing two cysteine residues that link the two heavy chains via disulfide bonds creating a F(ab')₂ molecule which is bivalent for antigen binding.

The lower hinge and beginning of the CH2 region, P-A-P-E-F/L-L-G-G-P-S-V-F (residues 5-16 of SEQ ID NO: 1 and 2) comprises cleavage sites for matrixmetalloproteinases, MMP-3 and MMP-12. Pepsin and MMP-7 also cleave in this region (P-A-P-E-L*L-G). In addition, a group of physiologically relevant enzymes; neutrophil elastase (HNE), stromelysin (MMP-3) and macrophage elastase (MMP-12) cleave IgG at several positions to generate subtly different F(ab')₂, Fab and Fc fragments (see Table 1).

### Biological Characterization of Glycoform Variants

Fc-containing proteins can be compared for functionality by several well-known in vitro assays. In particular, affinity for members of the FcγRI, FcγRII, and FcγRIII family of Fcγ receptors is of interest. These measurements could be made using recombinant soluble forms of the receptors or cell-associated forms of the receptors. In addition, affinity for FcRn, the receptor responsible for the prolonged circulating half-life of IgGs, can be measured, for example, by BIAcore using recombinant soluble FcRn. Cell-based functional assays, such as ADCC assays and CDC assays, provide insights into the likely functional consequences of particular variant structures. In one embodiment, the ADCC assay is configured to have NK cells be the primary effector cell, thereby reflecting the functional effects on the FcγRIIIA receptor. Phagocytosis assays may also be used to compare immune effector functions of different variants, as can assays that measure cellular responses, such as superoxide or inflammatory mediator release. In vivo models can be used as well, as, for example, in the case of using variants of anti-CD3 antibodies to measure T cell activation in mice, an activity that is dependent on Fc domains engaging specific ligands, such as Fcγ receptors.

### Protein Production Processes

Different processes involved with the production of Fc-containing proteins can impact Fc oligosaccharide structure. In one instance, the host cells secreting the Fc-containing protein are cultured in the presence of serum, e.g., fetal bovine serum (FBS) that was not previously subjected to an elevated heat treatment (for example, 56°C for 30 minutes). This can result in Fc-containing protein that contains no, or very low amounts of, sialic acid, due to the natural presence in the serum of active sialidase enzymes that can remove sialic acid from the Fc-containing proteins secreted from those cells. In another embodiment, the cells secreting the Fc-containing protein are cultured either in the presence of serum that was subjected to an elevated heat treatment, thereby inactivating sialidase enzymes, or in the absence of serum or other medium components that may contain sialidase enzymes, such that the Fc-containing protein has higher or lower levels of glycosylation or glycosylation variants.

In another embodiment, the conditions used to purify and further process Fc-containing proteins are established that will favor optimal glycan content. In one embodiment, the conditions produce maximal or minimal oligosaccharide content or cause the transformation of the expressed Fc-containing polypeptide in a predominant glycoform. For example, because sialic acid is acid-labile, prolonged exposure to a low pH environment, such as following elution from protein A chromatography column or viral inactivation efforts, may lead to a reduction in sialic acid content. In another embodiment, the glycosylated material is subjected to chromatography using a lectin-immobilized support material which will selectively bind or retard the passage of proteins displaying specific saccharides or oligosaccharide complexes. In the case of immobilized-lection column, the nonbinding flow-through (T, through) or the column unbound fraction can be separated from the bound fraction (B, bound), the latter collected while passing elution buffer through the column. It may also be possible to separately collect a weakly bound fraction or the column retarded fraction (R, retarded), for example, by collecting Fc-containing protein that elutes during continued washing of the column with the original sample buffer. Examples of lectins that may enrich for sialylated or asialylated Fc-containing proteins are the lectin from *Maackia amurensis* (MAA), which specifically binds oligosaccharides with terminal sialic acid, and the lectin wheat germ agglutinin (WGA), which specifically binds oligosaccharides with either terminal sialic acid or terminal N-acetylglucosamine (GlcNAc). Another example is the lectin Ricin I (RCA), which binds oligosaccharides with terminal galactose. In the latter example, the non-binding flow-through fraction may be enriched for sialylated Fc-containing molecules. Other lectins known in the art include those provided by Vector labs and EY labs.

### Host Cell Selection or Host Cell Engineering

As described herein, the host cell chosen for expression of the recombinant Fc-containing protein or monoclonal antibody is an important contributor to the final composition, including, without limitation, the variation in composition of the oligosaccharide moieties decorating the protein in the immunoglobulin CH2 domain. Thus, one aspect of the invention involves the selection of appropriate host cells for use and/or development of a production cell expressing the desired therapeutic protein.

In one embodiment in which the sialic acid content of the antibody or Fc-fusion is diminished, the host cell is a cell that is naturally deficient or devoid of sialyltransferases. In another embodiment, the host cell is genetically modified to be devoid of sialyltransferases. In a further embodiment, the host cell is a derivative host cell line selected to express reduced or undetectable levels of sialyltransferases. In yet another embodiment, the host cell is naturally devoid of, or is genetically modified to be devoid of, CMP-sialic acid synthetase, the enzyme that catalyzes the formation of CMP-sialic acid, which is the source of sialic acid used by sialyltransferase to transfer sialic acid to the antibody. In a related embodiment, the host cell may be naturally devoid of, or is genetically modified to be devoid of, pyruvic acid synthetase, the enzyme that forms sialic acid from pyruvic acid.

In an additional embodiment, the host cell may be naturally devoid of, or is genetically modified to be devoid of, galactosyltransferases, such that antibodies expressed in said cells lack galactose. Without galactose, sialic acid will not be attached. In a separate embodiment, the host cell may naturally overexpress, or be genetically modified to overexpress, a sialidase enzyme that removes sialic acid from antibodies during production. Such a sialidase enzyme may act intracellularly on antibodies before the antibodies are secreted or be secreted into the culture medium and act on antibodies that have already been secreted into the medium and may further contain a galactase. Methods of selecting cell lines with altered glycosylases and which express glycoproteins with altered carbohydrate compositions have been described (Ripka and Stanley, 1986. Somatic Cell Mol Gen 12:51-62; US2004/0132140). Methods of engineering host cells to produce antibodies with altered glycosylation patterns resulting in enhanced ADCC have been taught in, e.g., U.S. Pat. 6,602,864, wherein the host cells harbor a nucleic acid encoding at least one glycoprotein modifying glycosyl transferase, specifically β (1,4)-N-acetylglucosaminyltranferase III (GnTIII).

Other approaches to genetically engineering the glycosylation properties of a host cell through manipulation of the host cell glycosyltransferase involve eliminating or suppressing the activity, as taught in EP1,176,195, specifically, alpha1,6 fucosyltransferase (FUT8 gene product). It would be known to one skilled in the art to practice the methods of host cell engineering in other than the specific examples cited above. Further, the engineered host cell may be of mammalian origin or may be selected from COS-1, COS-7, HEK293, BHK21, CHO, BSC-1, Hep G2, 653, SP2/0, 293, HeLa, myeloma, lymphoma, yeast, insect or plant cells, or any derivative, immortalized or transformed cell thereof.

In another embodiment, the method of suppressing or eliminating the activity of the enzyme required for oligosaccharide attachment may be selected from the group consisting of gene silencing, such as by the use of siRNA, genetic knock-out, or addition of an enzyme inhibitor, such as by co-expression of an intracellular antibody or peptide specific for the enzyme that binds and blocks its enzymatic activity, and other known genetic engineering techniques. In another embodiment, a method of enhancing the expression or activity of an enzyme that blocks saccharide attachment, or a saccharidase enzyme that removes sugars that are already attached, may be selected from the group consisting of: transfections with recombinant enzyme genes, transfections of transcription factors that enhance enzyme RNA synthesis, and genetic modifications that enhance stability of enzyme RNA, all leading to enhanced activity of enzymes, such as sialidases, that result in lower levels of sialic acid in the purified product. In another embodiment, specific enzyme inhibitors may be added to the cell culture medium. Alternatively, the host cell may be selected from a species or organism incapable of glycosylating polypeptides, e.g. a prokaryotic cell or organism, such as and of the natural or engineered *E. coli spp*, *Klebsiella spp*., or *Pseudomonas spp.*

### Antibodies

An antibody described in this application can include or be derived from any mammal, such as, but not limited to, a human, a mouse, a rabbit, a rat, a rodent, a primate, a goat, or any combination thereof and includes isolated human, primate, rodent, mammalian, chimeric, humanized and/or CDR-grafted antibodies, immunoglobulins, cleavage products and other specified portions and variants thereof.

The antibodies, Fc-comprising proteins, or Fc fragments described herein can be derived in several ways well known in the art. In one aspect, the antibodies are conveniently obtained from hybridomas prepared by immunizing a mouse or other animal with the target peptides, cells or tissues extracts. The antibodies can thus be obtained using any of the hybridoma techniques well known in the art, see, e.g., Ausubel, et al., ed., Current Protocols in Molecular Biology, John Wiley & Sons, Inc., NY, NY (1987-2001); Sambrook, et al., Molecular Cloning: A Laboratory Manual, 2nd Edition, Cold Spring Harbor, NY (1989); Harlow and Lane, antibodies, a Laboratory Manual, Cold Spring Harbor, NY (1989); Colligan, et al., eds., Current Protocols in Immunology, John Wiley & Sons, Inc., NY (1994-2001); Colligan et al., Current Protocols in Protein Science, John Wiley & Sons, NY, NY, (1997-2001).

The antibodies or Fc-fusion proteins or components and domains thereof may also be obtained from selecting from libraries of such domains or components, e.g., a phage library. A phage library can be created by inserting a library of random oligonucleotides or a library of polynucleotides containing sequences of interest, such as from the B-cells of an immunized animal or human (Smith, G.P. 1985. Science 228: 1315-1317). Antibody phage libraries contain heavy (H) and light (L) chain variable region pairs in one phage allowing the expression of single-chain Fv fragments or Fab fragments (Hoogenboom, et al. 2000, Immunol. Today 21(8) 371-8). The diversity of a phagemid library can be manipulated to increase and/or alter the immunospecificities of the monoclonal antibodies of the library to produce and subsequently identify additional, desirable, human monoclonal antibodies. For example, the heavy (H) chain and light (L) chain immunoglobulin molecule encoding genes can be randomly mixed (shuffled) to create new HL pairs in an assembled immunoglobulin molecule. Additionally, either or both the H and L chain encoding genes can be mutagenized in a complementarity determining region (CDR) of the variable region of the immunoglobulin polypeptide, and subsequently screened for desirable affinity and neutralization capabilities. Antibody libraries also can be created synthetically by selecting one or more human framework sequences and introducing collections of CDR cassettes derived from human antibody repertoires or through designed variation (Kretzschmar and von Ruden 2000, Current Opinion in Biotechnology, 13:598-602). The positions of diversity are not limited to CDRs, but can also include the framework segments of the variable regions or may include other than antibody variable regions, such as peptides. Other libraries of target binding components which may include other than antibody variable regions are ribosome display, yeast display, and bacterial displays. Ribosome display is a method of translating mRNAs into their cognate proteins while keeping the protein attached to the RNA. The nucleic acid coding sequence is recovered by RT-PCR (Mattheakis, L.C. et al. 1994. Proc. Natl. Acad. Sci. USA 91, 9022). Yeast display is based on the construction of fusion proteins of the membrane-associated alpha-agglutinin yeast adhesion receptor, aga1 and aga2, a part of the mating type system (Broder, et al. 1997. Nature Biotechnology, 15:553-7). Bacterial display is based on fusion of the target to exported bacterial proteins that associate with the cell membrane or cell wall (Chen and Georgiou 2002. Biotechnol Bioeng, 79:496-503). In comparison to hybridoma technology, phage and other antibody display methods afford the opportunity to manipulate selection against the antigen target in vitro and without the limitation of the possibility of host effects on the antigen or vice versa.

Herein also disclosed are nucleic acids encoding the compositions as isolated polynucleotides or as portions of expression vectors including vectors compatible with prokaryotic, eukaryotic or filamentous phage expression, secretion and/or display of the compositions or directed mutagens thereof.

### Use of the Fc-Containing Molecules

The compositions (antibody, Fc-fusions, Fc fragments) generated by any of the above described methods may be used to diagnose, treat, detect, or modulate human disease or specific pathologies in cells, tissues, organs, fluid, or, generally, a host. As taught herein, modification of glycosylation of the Fc portion of an antibody, Fc-fusion protein, or Fc fragment to resist proteolytic digestion by proteases known to be present in a fluid, compartment, tissue or organ that is the target of treatment can be used to produce therapeutic molecules; these molecules may retain their original targeting properties and will be less prone to degradation by these proteases.

The protease, the cleavage activity of which is to be resisted, is selected from the group consisting of pepsin, plasmin, trypsin, chymotrypsin, a matrix metalloproteinase, a serine endopeptidase, and a cysteine protease, arising from the host or a pathogen which may be a parasite, bacterium or a virus. In a specific embodiment, the protease is a matrix metalloproteinase selected from the group consisting of gelatinase A (MMP2, gelatinase B (MMP-9), matrix metalloproteinase-7 (MMP-7), stromelysin (MMP-3), and macrophage elastase (MMP-12). The modifications for alteration of glycosylation of the Fc portion of the molecule or Fc molecule (using EU numbering), can be selected from removal of a glycosylation site in the CH2 domain (substitution of Asn 297), addition of an N-linked glycosylation site in the CH3 domain by substituting an Asn at 359 and a Thr at 361, addition of an N-linked glycosylation site in the CH3 domain by substituting an Asn at 382 and a Thr at 384, and addition of an N-linked glycosylation site in the CH3 domain by substituting an Asn at 419 and a Thr at 421. The addition of glycosylation sites to the CH3 domain are expected to increase the volume of hydration of the resulting molecule and increase persistence in the body.

The diseases or pathologies that may be amenable to treatment using a composition provided by the invention include, but are not limited to: cancer or proliferative disease, inflammatory or rheumatic diseases, autoimmune disorders, neurological disorders, fibrosis, cardiovascular disease, dermatological and infectious disease, and conditions resulting from burns or injury.

Cancer or proliferative disorders which are amenable to treatment with the compositions of the invention are selected from solid tumors, metastatic tumors, liquid tumors, and benign tumors, such as lymphomas, lymphoblastic or myelogenous leukemia, (ALL), B-cell, T-cell or FAB ALL, acute myeloid leukemia (AML), chronic myelocytic leukemia (CML), chronic lymphocytic leukemia (CLL), hairy cell leukemia, myelodyplastic syndrome (MDS), a lymphoproliferative disease, Hodgkin's disease, Castleman's disease, a malignant lymphoma, non-Hodgkin's lymphoma, Burkitt's lymphoma, multiple myeloma, Kaposi's sarcoma, colorectal carcinoma, pancreatic carcinoma, renal cell carcinoma, breast cancer, nasopharyngeal carcinoma, malignant histiocytosis, adenocarcinomas, squamous cell carcinomas, sarcomas, malignant melanoma, particularly metastatic melanoma, and hemangioma.

Inflammatory or immune-system mediated diseases which are amenable to treatment with the compositions of the invention are selected from rheumatoid arthritis, juvenile rheumatoid arthritis, systemic onset juvenile rheumatoid arthritis, psoriasis, psoriatic arthritis, ankylosing spondilitis, gastric ulcer, arthropathies and arthroscopic plaque, osteoarthritis, inflammatory bowel disease, ulcerative colitis, systemic lupus erythematosis, antiphospholipid syndrome, uveitis, optic neuritis, idiopathic pulmonary fibrosis, systemic vasculitis, Wegener's granulomatosis, sarcoidosis, orchitis, allergic and atopic diseases, asthma and atopic asthma, allergic rhinitis, eczema, allergic contact dermatitis, allergic conjunctivitis, hypersensitivity pneumonitis, organ transplant rejection, graft-versus-host disease, and systemic inflammatory response syndrome.

Other diseases or conditions which are amenable to treatment with the compositions of the invention are pemphigus, scleroderma, chronic obstructive pulmonary disease, infections of gram negative or gram positive bacteria, viral infections such as influenza and HIV, infection with parasites such as malaria or leishmaniasis, leprosy, encephalitis, Candidiasis, amyloidosis, Alzheimer's disease, myocardial infarction, congestive heart failure, stroke, ischemic stroke, and hemorrhage.

As specifically exemplified herein, adding an N-linked glycan in the CH3 domain of an Fc region (by substituting an Asn residue at 359 and a Thr residue at 361(EU numbering)) a compound which is a peptide Fc-fusion protein is made less sensitive to a matrix metalloproteinase (MMP-3) and a serine endopeptidase (NE) while maintaining the FcRn binding affinity of the molecule and the ADCC/CDC activity.

While having described the invention in general terms, the embodiments of the invention will be further disclosed in the following examples that should not be construed as limiting the scope of the claims.

### EXAMPLE 1. CONSTRUCTION OF Fc GLYCOSYLATION VARIANTS

Experimentation was performed on the EMP-1 Fc fusion (CNTO530) described as an EPO MIMETIBODY™ construct (Fc fusion) in U.S. Pat. 7,393,662 (SEQ ID NO: 88) and a GLP-1 MIMETIBODY™ construct (Fc fusion) (CNTO736) described in WO/05097175. Both constructs contain an Fc region derived from a human IgG4 antibody as shown in Fig. 1.

### CNTO 530 variants

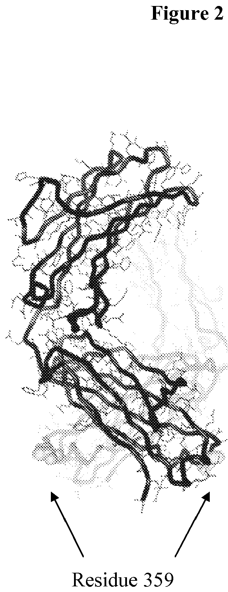

The plasmid encoding CNTO530, p2630, was used as the starting material to prepare NEM2631 using standard recombinant PCR and cloning methods. To introduce the T359N/N361T substitutions into EPO MIMETIBODY™ construct CNTO 530, a CH3-encoding restriction fragment was isolated fromNEM 2631 T359N/N361T plasmid p3051 and cloned in place of the corresponding fragment in plasmid p2630 encoding CNTO 530. The resulting plasmid, p3201, encoded the protein CNTO 530 T359N/N361T, herein referred to as CNTO 5303 (see Figure 2 and Table 1).

To prepare a CNTO 530 variant having the same T359N/N361T substitutions but lacking the native Fc glycosylation at position 297, the appropriate portion of plasmid p3201 was PCR-amplified with mutagenic oligonucleotides and cloned to result in a T299N codon substitution (i.e., changed from ₂₉₇NST₂₉₉ to ₂₉₇NSN₂₉₉). The resulting plasmid was p3576 encoding the protein CNTO 530 T359N/N361T/T299N, herein referred to as CNTO 5304.

### CNTO 736 variants

To introduce the T359N/N361T substitutions into CNTO 736, a CH3-encoding restriction fragment was isolated from NEM 2631 T359N/N361T plasmid p3051 and cloned in place of the corresponding fragment in plasmid p2538 encoding CNTO 736. The resulting plasmid was p3349 encoding CNTO 736 T359N/N361T, herein referred to as CNTO 7363 (Table 1).

To prepare a CNTO 736 variant having the same T359N/N361T substitutions but lacking the native Fc glycosylation at position 297, the appropriate portion of plasmid p3349 was PCR-amplified and cloned to result in a T299N codon substitution. The resulting plasmid was p3577 encoding the protein CNTO 736 T359N/N361T/ T299N, herein referred to as CNTO 7364.

**Table 1.**

| Plasmid | Code | Description | Host | Code | mg/L* |
|---|---|---|---|---|---|
| p3201 | CNTO 5303 | CNTO 530 T359N/N361T | CHO | C1514A | 28 |
| p3576 | CNTO 5304 | CNTO 530 T359N/N361T/T299N | NS0 | C1670A | 20 |
| | | | | | |
| p3349 | CNTO 7363 | CNTO 736 T359N/N361T | NS0 | C1528A | 5-8 |
| p3577 | CNTO 7364 | CNTO 736 T359N/N361T/T299N | NS0 | C1671A | 5-8 |

| | | | | | |
|---|---|---|---|---|---|
| *observed production levels from transfected cells | | | | | |

*Expression and purifications*. CHO-K1SV cells (C1013A) were stably transfected with p3201 plasmid encoding CNTO 5303, resulting in isolation of CNTO 5303-producing cell line C1514A. Mouse NS0 cells were stably transfected with the above-described plasmids encoding CNTO 5304, CNTO 7363, and CNTO 7364, resulting in isolation of transfected cell lines C1670A, C1528A, and C1671A, respectively (Table 1). All four MIMETIBODY™ construct variants were purified from transfected cell supernatant by standard protein A chromatography. Since protein A and FcRn both bind in at the CH2-CH3 junction of the Fc domain, the successful purification using protein A columns suggested that the new glycosylation sites may not affect binding to FcRn (see below).

### EXAMPLE 2. CHARACTERIZAION OF THE Fc GLYCOSYLATION VARIANTS

A series of analytical, biophysical, and bioactivity tests were performed on the expressed constructs of Example 1.

MALDI-TOF-MS analyses were performed to characterize the glycan structures of the MIMETIBODY™ construct variants and to establish what proportion of the heavy chains were glycosylated at the new site.

MALDI-TOF-MS analyses of intact MIMETIBODY™ constructs indicated that the CNTO 5303, CNTO 5304, CNTO 7363, and CNTO 7364 samples were 75 - 95% occupied with glycan at the new site. Glycan analysis of these samples showed that they were more heterogeneous than the CNTO 530 and CNTO 736 MIMETIBODY™ construct glycans, with glycans at position 359 containing bi-, tri- and tetra-antennary structures. The native Fc glycans at position 297 in CNTO 5303 and CNTO 7363 were of the same structures as the native Fc glycans in CNTO 530 and CNTO 736, respectively, with the only observed difference being somewhat greater galactosylation in the original MIMETIBODY™ construct (e.g., about 50% G0 for CNTO 530 v. about 70% for CNTO 5303).

### Size and mobility was analyzed by SDS-PAGE

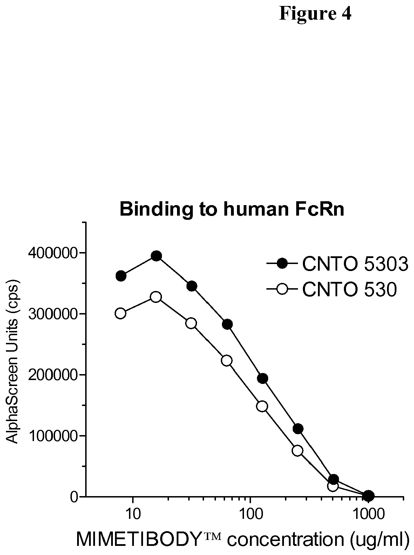

Purified CNTO 530, CNTO 5303, and CNTO 5304 were analyzed by SDS-PAGE by loading 1 ug/lane onto a 1.0 mm-thick BisTris 4-12% gradient gel under non-reducing conditions, and running the fractionation in MOPS SDS running buffer at 200V for 50 min. The gel was stained with coomassie G250 (SimplyBlue Safe Stain, Invitrogen), and the resulting images captured using an Alphalmager 2200 imaging system (Alpha Innotech) (Fig. 4).

The observed migrations in the SDS gel were in line with expectations, i.e., CNTO 5303 with a total of 4 N-glycosylation sites migrated more slowly, the apparent molecular weight increased by 3-4 kDa, than CNTO 530 and CNTO 5304 with 2 N-glycosylation sites. CNTO 5304 appeared to migrate slower than CNTO 530 despite having the same number of glycosylation sites. This is due to the new glycosylation site on CNTO 5304 having, relative to the native glycosylation on CNTO 530, a greater level of galactosylation and sialylation, as well as more tri-antennary and tetra-antennary structures. The molecular weight estimates are 57.5, 61.5, and 59.5 kDa for CNTO 530, CNTO 5303, and CNTO 5304, respectively.

### Bioactivity assessed by FcRn binding analyses.

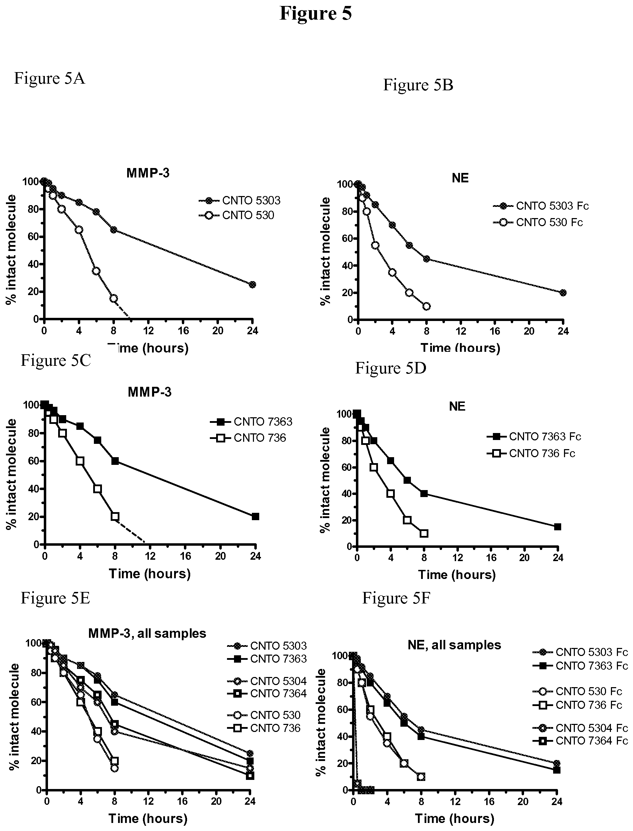

Because one factor in choosing where to introduce the new glycosylation was a wish to avoid the FcRn binding region, the binding to FcRn by CNTO 5303 was compared to CNTO 530 using AlphaScreen. The two MIMETIBODY™ construct samples were first dialyzed overnight at 4°C into pH 6.0 assay buffer (0.05M MES, 0.025% BSA, 0.001% Tween 20, pH 6.0) using Slide-A-Lyzer MINI dialysis units (10K MWCO; Thermo Scientific (Pierce)) as per package instructions. Antibody concentrations were determined by OD₂₈₀. The following components were then co-incubated in a 96-well, half-area, flat bottom, non-binding, white polystyrene assay plate with mixing for 1 hour at room temperature: biotinylated human IgG1 mAb (CNTO 6234; final concentration of 4 µg/ml), serially-diluted test samples, polyhistidine-tagged human FcRn (final concentration of 8 µg/ml), AlphaScreen nickel chelate acceptor beads (final concentration of 100 µg/ml), and AlphaScreen streptavidin-coated donor beads (final dilution of 1:250). All materials were diluted using assay buffer as described above. After incubation, plates were read on the EnVision instrument using the AlphaScreen protocol. The results (Fig. 5) showed that CNTO 5303 bound FcRn with similar affinity (K_{D} only 2-fold weaker than CNTO 530), indicating that FcRn binding was preserved in CNTO 5303.

### Protease-sensitivity evaluation.

The purified MIMETIBODY™ molecules were then evaluated for their relative sensitivity to two human proteases, recombinant matrix metalloproteinase-3 (MMP-3) and neutrophil elastase (NE). The MMP-3, believed to cleave after the ₂₂₈SCPAP sequence in the lower hinge, had been prepared at Centocor by transient expression in HEK cells as polyHis-tagged pro-MMP-3, purification by Talon affinity column, and frozen in aliquots. Human NE, which normally cleaves primarily after the ₂₂₀CDKT upper hinge sequence, but also can cleave at a secondary site in the lower hinge (see below), was obtained from Athens Research and Technologies (Athens, GA).

### Protease Sensitivity

*MMP-3.* Frozen MMP-3 was thawed, and then activated by incubating at 55°C for 25 minutes prior to performing MMP-3 digestions. Purified MIMETIBODY™ construct samples at ∼1 mg/ml were treated at 37°C with activated MMP3 (1:50, w/w) in 20 mM Tris-HCl buffer, pH 7.0, containing 2 mM calcium chloride. Aliquots (∼ 2 µl) were withdrawn at fixed time intervals (0, 0.5, 1, 2, 4, 6, 8 and 24 hrs) and were immediately mixed with 2 µl of matrix solution (the matrix solution was prepared by dissolving 10 of mg Sinapic acid in 1.0 ml 50% acetonitrile in water containing 0.1% trifluoroacetic acid). Two µl of this solution was loaded onto the MALDI target plate and allowed to air dry prior to mass spec analysis described below.

*Neutrophil Elastase.* Because the N-terminal peptide portions of the MIMETIBODY™ constructs were extremely sensitive to NE digestion (thereby complicating quantitations of intact molecules and interfering with focus on hinge-Fc resistance), and because a secondary NE cleavage site was observed to exist somewhere in the lower hinge region, especially in nonglycosylated IgGs, papain-generated Fc fragments were first prepared from each MIMETIBODY™ construct sample. Those Fc fragments from each MIMETIBODY™ construct, while at a concentration of ∼1 mg/ml, were treated at 37°C with NE (1:50, w/w) in 20 mM Tris-HCl buffer, pH 7.0. Aliquots (∼ 2 µl) were withdrawn at fixed time intervals (0, 0.5, 1, 2, 4, 6, 8 and 24 hrs) and were immediately mixed with 2 µl of matrix solution (the matrix solution was prepared by dissolving 10 of mg Sinapic acid in 1.0 ml 50% acetonitrile in water containing 0.1% trifluoroacetic acid). Two µl of this solution was loaded onto the MALDI target plate and allowed to air dry prior to mass spec analysis.

The IgG and IgG fragments in the proteolytic digest were analyzed using MALDI-TOF-MS Analysis. MALDI-TOF-MS analyses were carried out using a Voyager DE Biospectrometry workstation (Applied BioSystems, Foster City, CA) in linear or reflectron positive ion ([M + H]⁺) mode with delayed extraction. The instrument was externally calibrated with a protein calibration kit (Sigma).The results showed that the presence of N-linked glycosylation at Asn359 clearly conferred greater resistance to both MMP-3 (Figures 5A, 5C, 5E) and NE (Figure 5B, 5D, 5F), two proteases that cleave these substrates in the lower hinge region. After an 8-hour incubation with MMP-3, less than 20% of the original CNTO 530 remained intact, whereas more than 60% of CNTO 5303 remained intact. Similar results were observed with CNTO 7363 and CNTO 736. After an 8-hour incubation with NE, less than 10% of CNTO 530 Fc was intact, whereas 50% of CNTO 5303 Fc was intact - and similar results were again observed with Fc fragments from CNTO 7363 and CNTO 736. The CNTO 5304 and CNTO 7364 variants that had the new glycosylation at position 359, but lacked the native Fc glycosylation at 297 showed intermediate sensitivity to MMP-3 (Figure 5E) but markedly greater sensitivity to NE (Figure 5F). It remains to be determined to what extent NE sensitivity is directly influenced by the lack of native Fc glycosylation or indirectly by the resulting mis-folding of the upper Fc domain in the absence of native glycosylation. Because both MMP-3 and NE cleave in the vicinity of the MIMETIBODY™ construct hinge region, the new glycosylation site introduced far from the cleavage sites (see Figure 2) apparently has allosteric effects on protein conformation, as observed with some amino acid substitutions. However, it cannot be ruled out that the T359N or N361T substitutions themselves might result in such an allosteric effect.

### EXAMPLE 3. IN VIVO BEHAVIOR OF THE Fc GLYCOSYLATION VARIANTS

In this study, the pharmacokinetics of the glycosylation variants of CNTO530 were compared in mice. Normal, healthy female Balb/c mice, 8-12 weeks old (approximately 18-22 g) from Charles Rivers Laboratories (Raleigh, NC) were randomized by weight and group-housed (4 mice/cage) in plastic filter-topped cages and supplied with commercial rodent chow and acidified water *ad lib.* Mice (4 per test article) were injected intraperitoneally with a 10 ml/kg dose of either CNTO 530 or CNTO 5303 formulated in Dulbecco's PBS at 0.1 mg/ml in order to achieve a dose of 1 mg/kg.

Blood samples were collected on days 2, 7, 16, 26, and 35 by serial retro-orbital bleeds from each CO₂-anesthetized mouse during the first 26 days. Terminal blood samples were collected on day 35 via cardiac puncture from CO₂-anesthetized mice. All samples were marked as to the animal it derived from so that time course analyses could be performed on each individual animal.

All blood samples were allowed to stand at room temperature for at least 30 minutes, but no longer than 1 hour, centrifuged at 3500 rpm for 15 minutes and the serum separated. The serum samples were stored at -20°C until the end of the study, at which time all samples were analyzed together.

Serum samples from all mice were analyzed for human Fc by a standard ELISA entailing coating 96-well EIA plates with polyclonal goat anti-human IgG Fc fragment, incubating varying dilutions of the serum samples, and detecting bound human IgG with HRP-conjugated polyclonal goat anti-human IgG followed by addition of the appropriate color substrates. Titrated amounts of test article spiked into normal sera were used to establish a standard curve for quantitation purposes.

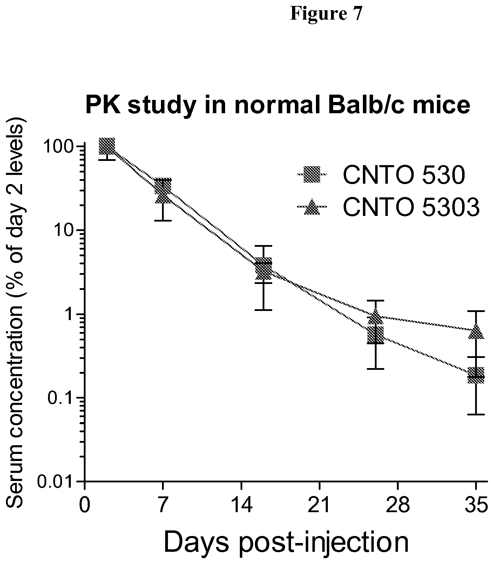

The concentrations of human Fc determined for each serum sample were normalized to the day 2 serum levels and graphed. The results revealed that the pharmacokinetic profile of the CNTO 5303 glycosylation variant was essentially indistinguishable from that of CNTO 530, indicating that the novel glycans did not have a deleterious effect on half-life in normal, healthy mice (Fig. 7).

## Claims

1. An Fc-containing molecule comprising a modified antibody Fc domain amino acid sequence with N-glycosylation sites at the ends of loop structures in the CH3 domain, wherein the Fc-containing molecule has increased resistance to protease, as compared to Fc-containing molecules having the analogous unmodified antibody Fc domain amino acid sequence.

2. The Fc-containing molecule of claim 1, wherein:
(i) the Fc domain is from any of an IgG1, IgG2, IgG3, and IgG4 molecule; or
(ii) the Fc-containing molecule is an antibody or Fc fusion protein.

3. The Fc-containing molecule of claim 1 or claim 2, wherein the protease is selected from the group consisting of pepsin, plasmin, trypsin, chymotrypsin, a matrix metalloproteinase, a serine endopeptidase, and a cysteine protease, optionally wherein the protease is a matrix metalloproteinase selected from the group consisting of gelatinase A (MMP2), gelatinase B (MMP-9), matrix metalloproteinase-7 (MMP-7), stromelysin (MMP-3), and macrophage elastase (MMP-12).

4. The Fc-containing molecule of any preceding claim, wherein the Fc domain exhibits N-glycosylation sites correlative to EU numbering at at least one of residues 359, 382, and 419.

5. The Fc-containing molecule of claim 4, wherein:
(i) the Fc domain exhibits N-glycosylation sites correlative to EU numbering at residues 359, 382, and 419 of the Fc domain; or
(ii) the Fc domain exhibits N-glycosylation sites correlative to EU numbering at residues 359, 382, and 419 of the Fc domain, and an N-glycosylation site at residue 297 of the Fc domain is removed, optionally wherein residue 299 is changed from Thr to Asn, residue 359 is changed from Thr to Asn, residue 361 is changed from Asn to Thr, residue 419 is changed from Thr to Asn, and residue 421 is changed from Asn to Thr.

6. The Fc-containing molecule of claim 4, wherein the Fc domain has a change from wild-type at at least one of residue 359, 361, 419, and 421.

7. The Fc-containing molecule of claim 6, wherein:
(i) residue 359 is changed from Thr to Asn and residue 361 is changed from Asn to Thr, and/or residue 419 is changed from Thr to Asn and residue 421 is changed from Asn to Thr; or
(ii) the Fc domain has a change from wild-type at residues 359, 361, 419, and 421, optionally wherein residue 359 is changed from Thr to Asn, residue 361 is changed from Asn to Thr, residue 419 is changed from Thr to Asn, and residue 421 is changed from Asn to Thr.

8. The Fc-containing molecule of claim 1, wherein correlative to EU numbering at least one of residues 228, 234, and 235 in the hinge region is altered.

9. An Fc-containing molecule with increased resistance to protease according to claim 1, comprising an antibody Fc domain with N-glycosylation sites correlative to EU numbering at residues 359, 382, and 419 of the Fc domain and an N-glycosylation site at residue 297 of the Fc domain is removed, wherein the Fc domain has a change from wild-type at residues 299, 359, 361, 419, and 421.

10. The Fc-containing molecule of claim 9, wherein correlative to EU numbering at least one of residues 228, 234, and 235 in the hinge region is altered.

11. The Fc-containing molecule of any preceding claim, for use in a method for treating a disease associated with the presence of elevated levels of proteases.

12. A method of increasing resistance of an Fc-containing protein to cleavage by a protease, comprising adding N-glycosylation sites to the Fc-containing protein correlative to the EU numbering at at least one of positions 359, 382, and 419.

13. The method of claim 12, comprising adding N-glycosylation sites to the Fc-containing protein correlative to the EU numbering at positions 359, 382, and 419, optionally further comprising removing the N-glycosylation site correlative to the EU numbering at position 297.

14. The method of claim 12, further comprising altering from wild type correlative to EU numbering at least one of residues 228, 234, and 235 in the hinge region.

## Patentansprüche

1. Fc-haltiges Molekül, umfassend eine modifizierte Antikörper-Fc-Domäne-Aminosäuresequenz mit N-Glykosylierungsstellen an den Enden von Loop-Strukturen in der CH3-Domäne, wobei das Fc-haltige Molekül eine erhöhte Resistenz gegenüber Protease im Vergleich zu Fc-haltigen Molekülen mit der analogen unmodifizierten Antikörper-Fc-Domäne-Aminosäuresequenz aufweist.

2. Fc-haltiges Molekül nach Anspruch 1, wobei:
(i) die Fc-Domäne aus einem IgG1-, IgG2-, IgG3- oder IgG4-Molekül stammt; oder
(ii) es sich bei dem Fc-haltigen Molekül um ein Antikörper- oder Fc-Fusionsprotein handelt.

3. Fc-haltiges Molekül nach Anspruch 1 oder Anspruch 2, wobei die Protease aus der aus Pepsin, Plasmin, Trypsin, Chymotrypsin, einer Matrix-Metalloproteinase, einer Serin-Endopeptidase und einer Cystein-Protease bestehenden Gruppe ausgewählt ist, gegebenenfalls wobei es sich bei der Protease um eine aus der aus Gelatinase A (MMP2), Gelatinase B (MMP-9), Matrix-Metalloproteinase-7 (MMP-7), Stromelysin (MMP-3) und Makrophagen-Elastase (MMP-12) bestehenden Gruppe ausgewählte Matrix-Metalloproteinase handelt.

4. Fc-haltiges Molekül nach einem vorhergehenden Anspruch, wobei die Fc-Domäne N-Glykosylierungsstellen entsprechend EU-Nummerierung an wenigstens einem der Reste 359, 382 und 419 zeigt.

5. Fc-haltiges Molekül nach Anspruch 4, wobei:
(i) die Fc-Domäne N-Glykosylierungsstellen entsprechend EU-Nummerierung an Rest 359, 382 und 419 der Fc-Domäne zeigt; oder
(ii) die Fc-Domäne N-Glykosylierungsstellen entsprechend EU-Nummerierung an Rest 359, 382 und 419 der Fc-Domäne zeigt und eine N-Glykosylierungsstelle an Rest 297 der Fc-Domäne entfernt ist, gegebenenfalls wobei Rest 299 von Thr zu Asn, Rest 359 von Thr zu Asn, Rest 361 von Asn zu Thr, Rest 419 von Thr zu Asn und Rest 421 von Asn zu Thr geändert ist.

6. Fc-haltiges Molekül nach Anspruch 4, wobei die Fc-Domäne eine Änderung vom Wildtyp an wenigstens einem der Reste 359, 361, 419 und 421 aufweist.

7. Fc-haltiges Molekül nach Anspruch 6, wobei:
(i) Rest 359 von Thr nach Asn und Rest 361 von Asn nach Thr geändert ist und/oder Rest 419 von Thr nach Asn und Rest 421 von Asn nach Thr geändert ist; oder
(ii) die Fc-Domäne eine Änderung vom Wildtyp an Rest 359, 361, 419 und 421 aufweist, gegebenenfalls wobei Rest 359 von Thr nach Asn, Rest 361 von Asn nach Thr, Rest 419 von Thr nach Asn und Rest 421 von Asn nach Thr geändert ist.

8. Fc-haltiges Molekül nach Anspruch 1, wobei entsprechend EU-Nummerierung wenigstens einer der Reste 228, 234 und 235 in der Hinge-Region verändert ist.

9. Fc-haltiges Molekül mit erhöhter Resistenz gegenüber Protease gemäß Anspruch 1, umfassend eine Antikörper-Fc-Domäne mit N-Glykosylierungsstellen entsprechend EU-Nummerierung an Rest 359, 382 und 419 der Fc-Domäne, und eine N-Glykosylierungsstelle an Rest 297 der Fc-Domäne ist entfernt, wobei die Fc-Domäne eine Änderung vom Wildtyp an Rest 299, 359, 361, 419 und 421 aufweist.

10. Fc-haltiges Molekül nach Anspruch 9, wobei entsprechend EU-Nummerierung wenigstens einer der Reste 228, 234 und 235 in der Hinge-Region verändert ist.

11. Fc-haltiges Molekül nach einem vorhergehenden Anspruch, zur Verwendung bei einem Verfahren zur Behandlung einer mit dem Vorliegen erhöhter Spiegel von Proteasen assoziierten Krankheit.

12. Verfahren zur Erhöhung der Resistenz eines Fc-haltigen Proteins gegenüber Spaltung durch eine Protease, umfassend Hinzufügen von N-Glykosylierungsstellen zum Fc-haltigen Protein entsprechend der EU-Nummerierung an wenigstens einer der Positionen 359, 382 und 419.

13. Verfahren nach Anspruch 12, umfassend Hinzufügen von N-Glykosylierungsstellen zum Fc-haltigen Protein entsprechend der EU-Nummerierung an Position 359, 382 und 419, gegebenenfalls ferner umfassend Entfernen der N-Glykosylierungsstelle entsprechend der EU-Nummerierung an Position 297.

14. Verfahren nach Anspruch 12, ferner umfassend Verändern vom Wildtyp entsprechend EU-Nummerierung wenigstens eines der Reste 228, 234 und 235 in der Hinge-Region.

## Revendications

1. Molécule contenant Fc comprenant une séquence d'acides aminés de domaine Fc d'anticorps modifiée avec des sites de N-glycosylation aux extrémités de structures de boucle dans le domaine CH3, la molécule contenant Fc ayant une résistance aux protéases augmentée, par rapport à des molécules contenant Fc ayant la séquence d'acides aminés de domaine Fc d'anticorps non modifiée analogue.

2. Molécule contenant Fc de la revendication 1, dans laquelle :
(i) le domaine Fc est de l'un quelconque d'une molécule IgG1, IgG2, IgG3 et IgG4 ; ou
(ii) la molécule contenant Fc est une protéine de fusion d'anticorps ou Fc.

3. Molécule contenant Fc de la revendication 1 ou la revendication 2, la protéase étant choisie dans le groupe constitué de la pepsine, la plasmine, la trypsine, la chymotrypsine, une métalloprotéinase de matrice, une sérine endopeptidase, et une cystéine protéase, la protéase étant facultativement une métalloprotéinase de matrice choisie dans le groupe constitué de la gélatinase A (MMP2), la gélatinase B (MMP-9), la métalloprotéinase de matrice 7 (MMP-7), la stromélysine (MMP-3), et une élastase de macrophage (MMP-12).

4. Molécule contenant Fc de l'une quelconque des revendications précédentes, dans laquelle le domaine Fc présente des sites de N-glycosylation corrélés à la numérotation EU à au moins un des résidus 359, 382 et 419.

5. Molécule contenant Fc de la revendication 4, dans laquelle :
(i) le domaine Fc présente des sites de N-glycosylation corrélés à la numérotation EU aux résidus 359, 382 et 419 du domaine Fc ; ou
(ii) le domaine Fc présente des sites de N-glycosylation corrélés à la numérotation EU aux résidus 359, 382 et 419 du domaine Fc, et un site de N-glycosylation au résidu 297 du domaine Fc est enlevé, facultativement où le résidu 299 est changé de Thr en Asn, le résidu 359 est changé de Thr en Asn, le résidu 361 est changé de Asn en Thr, le résidu 419 est changé de Thr en Asn, et le résidu 421 est changé de Asn en Thr.

6. Molécule contenant Fc de la revendication 4, dans laquelle le domaine Fc présente un changement par rapport au type sauvage à au moins un des résidus 359, 361, 419 et 421.

7. Molécule contenant Fc de la revendication 6, dans laquelle :
(i) le résidu 359 est changé de Thr en Asn et le résidu 361 est changé de Asn en Thr, et/ou résidu 419 est changé de Thr en Asn et le résidu 421 est changé de Asn en Thr ; ou
(ii) le domaine Fc présente un changement par rapport au type sauvage aux résidus 359, 361, 419 et 421, facultativement où le résidu 359 est changé de Thr en Asn, le résidu 361 est changé de Asn en Thr, le résidu 419 est changé de Thr en Asn, et le résidu 421 est changé de Asn en Thr.

8. Molécule contenant Fc de la revendication 1, dans laquelle, de façon corrélée à la numérotation EU, au moins un des résidus 228, 234 et 235 dans la région de charnière est modifié.

9. Molécule contenant Fc ayant une résistance augmentée aux protéases selon la revendication 1, comprenant un domaine d'anticorps Fc avec des sites de N-glycosylation corrélés à la numérotation EU aux résidus 359, 382 et 419 du domaine Fc et un site de N-glycosylation au résidu 297 du domaine Fc est enlevé, où le domaine Fc présente un changement par rapport au type sauvage aux résidus 299, 359, 361, 419 et 421.

10. Molécule contenant Fc de la revendication 9, dans laquelle, de façon corrélée à la numérotation EU, au moins un des résidus 228, 234 et 235 dans la région de charnière est modifié.

11. Molécule contenant Fc de l'une quelconque des revendications précédentes, pour utilisation dans un procédé de traitement d'une maladie associée à la présence de taux élevés de protéases.

12. Procédé d'augmentation de la résistance d'une protéine contenant Fc au clivage par une protéase, comprenant l'ajout de sites de N-glycosylation à la protéine contenant Fc de façon corrélée à la numérotation EU à au moins une des positions 359, 382 et 419.

13. Procédé de la revendication 12, comprenant l'ajout de sites de N-glycosylation à la protéine contenant Fc de façon corrélée à la numérotation EU aux positions 359, 382 et 419, comprenant facultativement en outre l'élimination du site de N-glycosylation corrélé à la numérotation EU à la position 297.

14. Procédé de la revendication 12, comprenant en outre la modification par rapport au type sauvage corrélée à la numérotation EU d'au moins un des résidus 228, 234 et 235 dans la région de charnière.
